# EUROPEAN PATENT SPECIFICATION

(11) **EP 4 558 512 B1**
(45) Date of publication and mention of the grant of the patent: **11.02.2026**
(21) Application number: 23765305.0
(22) Date of filing: 19.07.2023
(51) Int. Cl.: C07K 7/02, A61P 31/04, A61K 38/00, C08L 75/02

(54) **LIPOOLIGOUREAS, PHARMACEUTICAL COMPOSITION, AND LIPOOLIGOUREAS FOR USE AS MEDICAMENT**
LIPOOLIGOHARNSTOFFE, PHARMAZEUTISCHE ZUSAMMENSETZUNG UND LIPOOLIGOHARNSTOFFE ZUR VERWENDUNG ALS MEDIKAMENT
LIPOOLIGOURÉES, COMPOSITION PHARMACEUTIQUE ET LIPOOLIGOURÉES DESTINÉS À ÊTRE UTILISÉS COMME MÉDICAMENT

(30) Priority: 19.07.2022 PL 44178922
(43) Date of publication of application: 28.05.2025
(73) Proprietor: Uniwersytet Warszawski, 00-927 Warszawa (PL)
(72) Inventor: SEK, Slawomir, 02-797 Warszawa (PL); PULKA-ZIACH, Karolina, 02-775 Warszawa (PL); JUHANIEWICZ-DEBINSKA, Joanna, 03-982 Warszawa (PL); BARTOSIK, Dariusz, 02-495 Warszawa (PL); LASEK, Robert, 02-304 Warszawa (PL); DZIUBAK, Damian, 04-950 Warszawa (PL); BACHURSKA-SZPALA, Paulina, 37-716 Orly (PL); BURDACH, Kinga, 02-758 Warszawa (PL)
(74) Representative: JD&P Patent Attorneys Joanna Dargiewicz & Partners
(86) International application number: PCT/IB2023/057366
(87) International publication number: WO 2024/018398

(56) References cited:
- VIOLETTE A ET AL: "Mimicking Helical Antibacterial Peptides with Nonpeptidic Folding Oligomers", CHEMISTRY & BIOLOGY, vol. 13, no. 5, 1 May 2006 (2006-05-01), pages 531 - 538, XP055307145, DOI: 10.1016/j.chembiol.2006.03.009
- BURDACH K ET AL: "Interactions of Linear Analogues of Battacin with Negatively Charged Lipid Membranes", MEMBRANES, vol. 11, no. 3, 192, 10 March 2021 (2021-03-10), XP093094654, DOI: 10.3390/membranes11030192

## Description

The present invention relates to lipooligoureas, pharmaceutical compositions containing lipooligoureas, and lipooligoureas for use as medicaments in the treatment of bacterial infections.

The increasing incidence of drug resistance in bacteria is now a significant clinical problem, hence the growing interest in compounds that can offer an effective alternative while exhibiting different mechanisms of action than the conventional antibiotics. A completely new group of compounds that can potentially meet these requirements are the lipooligoureas described below. Oligoureas are already known (see, for example, Violette et al., Chemistry & Biology 2006, 13(5), 531).

Commercially available conventional antibiotics in most cases exhibit specific effect based on disruption of biochemical processes occurring in bacterial cells. For example, they affect the activity of enzymes involved in cell wall synthesis, disrupt metabolic pathways or damage DNA. Peptide- and lipopeptide-based antibiotics are also known; their effects are less specific and based on mechanisms that use the ability of such compounds to penetrate and irreversibly destroy the structure of the bacterial cell membrane (see, for example, Alborn et al., Antimicrob. Agents Chemother. 1991, 35, 2282, and Kang et al., Journal of Microbiology vol. 2017, 55, 1). The membranolytic properties of antibiotic peptides and lipopeptides significantly reduce the likelihood of bacterial resistance to such compounds, precisely because of the nonspecific mechanism of action. Currently there are medicaments available on the market whose active antibiotic ingredients are, for example, lipopeptides. This class can include polymyxins, or daptomycin. The latter is a natural branched cyclic antibiotic lipopeptide of non-ribosomal origin. This lipopeptide exhibits potent bactericidal activity *in vitro* and *in vivo* against gram-positive bacteria that can cause serious and life-threatening diseases, as described, for example, in Tally, F.P. et al., "Daptomycin: a Novel Agent for Gram positive Infections," Exp. Opin. Invest. Drugs 8:1223-1238 (1999). Most lipopeptides described in the literature, like daptomycin, are of natural origin and are derived from bacteria or other organisms, which entails the cost of isolation from biological material. Moreover, most of the lipopeptides currently used in medicine, such as daptomycin or polymyxin, have cyclic, relatively complex structures, which in turn complicates potential synthesis and further modifications. However, examples are also known from the literature of synthetic compounds belonging to the lipopeptide group, which show significant potential as useful antibiotics and at the same time are based on non-cyclic, i.e. linear structures (see for example US 6,911,525; US 2015/0080292 A1; WO 2021/064593 A1).

The objective of the present invention is to provide a completely new class of compounds-lipooligoureas, with a non-cyclic structure, which exhibit antibacterial activity against gram-positive and gram-negative bacteria.

This objective has been achieved through the development of a new class of lipooligoureas with antibacterial properties.

Thus, the subject matter of the present invention is a lipooligourea of general formula 1

**FA-Phe^{u}-Xaa1^{u}-Xaa2^{u}-Leu^{u}-Xaa3^{u}-NH₂** **(1)**

wherein
FA is a linear or branched carboxylic acid residue containing 6 to 15 carbon atoms in the alkyl chain;
Xaa1^{u} is either Lys^{u} or Dab^{u} or Arg^{u};
Xaa2^{u} is either Lys^{u} or Dab^{u} or Leu^{u} or Arg^{u};
Xaa3^{u} is either Lys^{u} or Dab^{u} or Leu^{u} or Arg^{u}.

Preferably, the lipooligourea is selected from a group consisting of

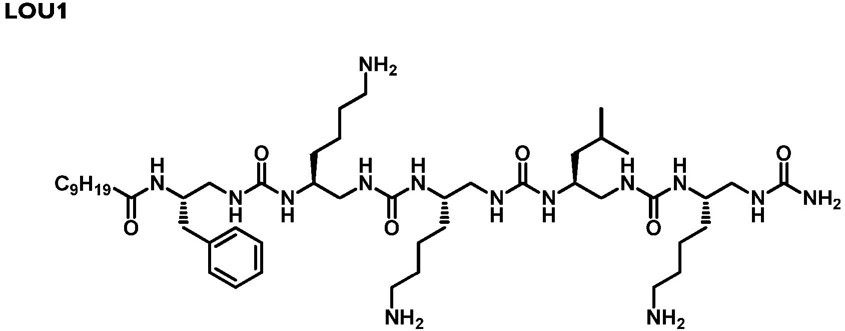

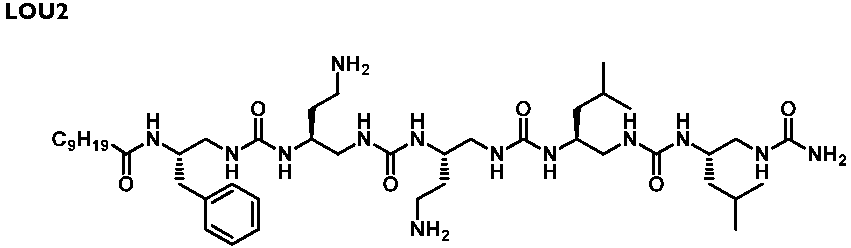

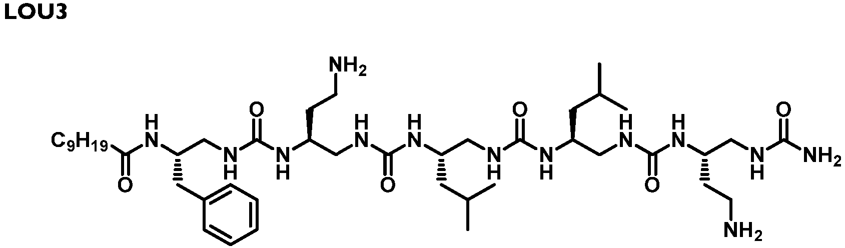

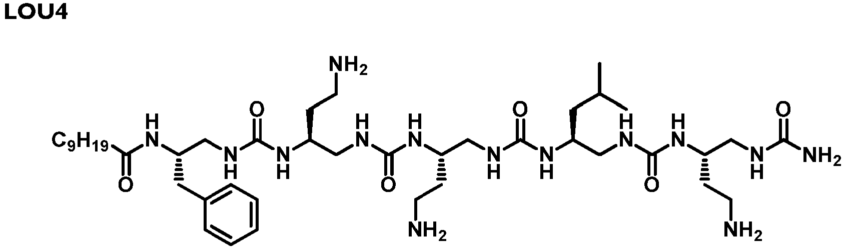

Subject matter of the invention is also the above-defined lipooligourea for use as a medicament.

Preferably, for use in the treatment or prevention of a bacterial infection.

Preferably, the bacterial infection is caused by either a gram-positive or a gram-negative bacteria.

Preferably, the bacterial infection is caused by a Staphylococcus bacteria.

Preferably, the bacterial infection is caused by *Staphylococcus aureus* or Staphylococcus *epidermidis.*

Preferably, the bacterial infection is caused by a strain of methicillin-resistant *Staphylococcus aureus* (MRSA).

The subject matter of the invention is a lipooligourea of formula LOU2 or LOU3 for use in the treatment or prevention of a bacterial infection caused by a strain of methicillin-resistant *Staphylococcus aureus* (MRSA).

Another subject matter of the invention is a pharmaceutical composition that contains a lipooligourea of the general formula 1

**FA-Phe^{u}-Xaa1^{u}-Xaa2^{u}-Leu^{u}-Xaa3^{u}-NH₂** **(1)**

wherein
FA is a linear or branched carboxylic acid residue containing 6 to 15 carbon atoms in the alkyl chain;
Xaa1^{u} is either Lys^{u} or Dab^{u} or Arg^{u};
Xaa2^{u} is either Lys^{u} or Dab^{u} or Leu^{u} or Arg^{u};
Xaa3^{u} is either Lys^{u} or Dab^{u} or Leu^{u} or Arg^{u}.

Preferably, the pharmaceutical composition contains a lipooligourea selected from the group consisting of

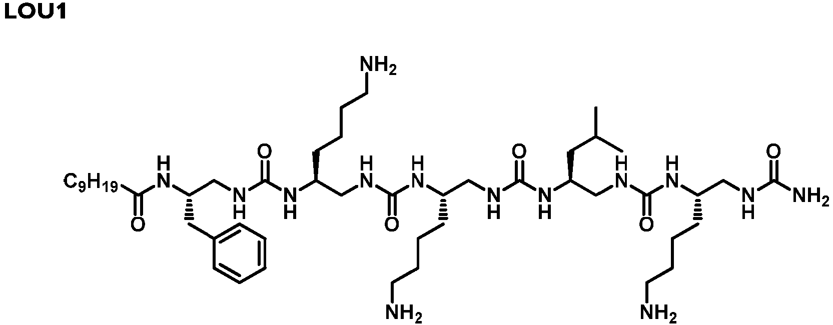

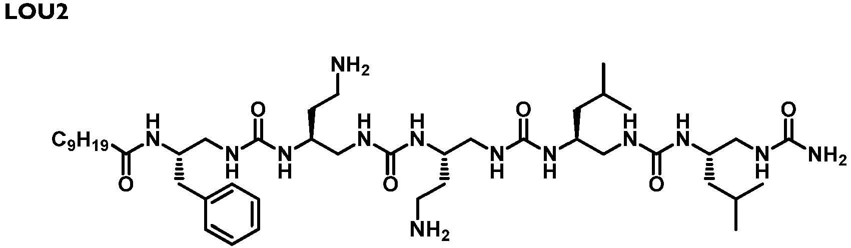

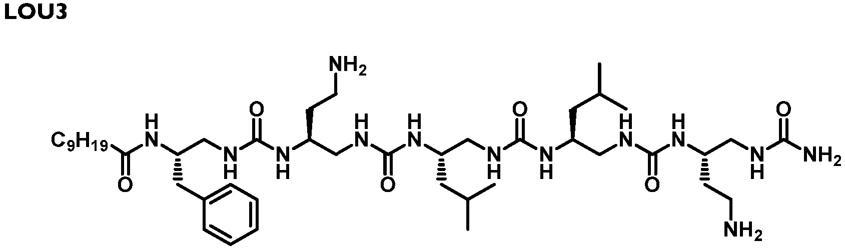

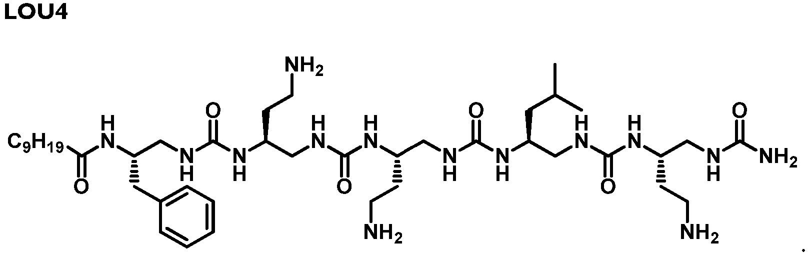

Preferably, the pharmaceutical composition contains 0.1 to 99% by weight of at least one lipooligourea.

Preferably, the pharmaceutical composition contains a pharmaceutically acceptable carrier and/or diluent and/or aid and/or excipient.

Preferably, the pharmaceutical composition contains corn starch and/or gelatin and/or lactose and/or sucrose and/or microcrystalline cellulose and/or kaolin and/or mannitol and/or dicalcium phosphate and/or sodium chloride and/or alginic acid.

Preferably, the pharmaceutical composition is in a form suitable for oral and/or intravenous and/or intramuscular and/or subcutaneous and/or parenteral and/or rectal and/or topical administration.

Preferably, the pharmaceutical composition is in the form of a tablet and/or capsule and/or elixir and/or suspension and/or syrup and/or gel and/or cream and/or ointment and/or suppository and/or aerosol and/or drops.

Preferably, the pharmaceutical composition further comprises at least one other antibacterial agent.

Unlike most conventional antibiotics, which, as mentioned earlier, act specifically, lipooligoureas show the ability to incorporate into and irreversibly damage the structure of the bacterial cell membrane. Since their activity is less specific compared to conventional medicaments, there is less likelihood of resistance to this type of compound. An important feature and advantage of lipooligoureas over antibiotic peptides and lipopeptides is also the lower susceptibility of oligourea chains to proteolytic degradation. This process can significantly reduce the activity of peptides and lipopeptides, while it is significantly eliminated in the case of synthetic peptidomimetics such as lipooligoureas. Thus, the solution presented herein relates to an entirely new class of antibacterial compounds, which are lipooligoureas. Said lipooligoureas consist of an oligourea portion comprising five urea residues linked to a linear or branched carboxylic acid residue. For the purposes of this description, standard nomenclature in the form of three-letter amino acid abbreviations with a "u" subscript indicating the urea nature of the residue is used to refer to the urea residues, which are analogs of individual amino acids. The structure of the described lipooligoureas can be represented by the general formula:

**FA-Phe^{u}-Xaa1^{u}-Xaa2^{u}-Leu^{u}-Xaa3^{u}-NH₂** **(1)**

wherein FA is a linear or branched carboxylic acid residue containing 6 to 15 carbon atoms in the alkyl chain;
Xaa1^{u} is either Lys^{u} or Dab^{u} or Arg^{u};
Xaa2^{u} is either Lys^{u} or Dab^{u} or Leu^{u} or Arg^{u};
Xaa3^{u} is either Lys^{u} or Dab^{u} or Leu^{u} or Arg^{u}.

The developed lipooligoureas have an amphiphilic structure, which allows their incorporation into the lipid bilayers, which constitute the cell membrane framework, and due to the presence of positively charged urea residues, such as Lys^{u}, Dab^{u} or Arg^{u}, they have an excess positive charge under physiological pH conditions, which further facilitates their interaction with the bacterial cell membrane, which usually has an excess negative charge due to its specific lipid composition (i.e. the presence of lipids from the phosphatidylglycerol group).

The pharmaceutical composition according to the invention contains any lipooligourea selected from the group of lipooligoureas of general formula **(1).** Preferably, the composition contains 0.1 to 99% by weight of a lipooligourea selected from the group comprising lipooligoureas of general formula **(1).** The pharmaceutical composition may contain pharmaceutically acceptable carriers and/or diluents and/or aids and/or excipients. For example, but without being limited to those mentioned, the pharmaceutical composition may contain corn starch and/or gelatin and/or lactose and/or sucrose and/or microcrystalline cellulose and/or kaolin and/or mannitol and/or dicalcium phosphate and/or sodium chloride and/or alginic acid. Preferably, the pharmaceutical composition is in a form suitable for oral and/or intravenous and/or intramuscular and/or subcutaneous and/or parenteral and/or rectal and/or topical administration (e.g. on the skin, into the nose, into the ears), e.g., in the form of tablets and/or capsules and/or elixir and/or suspension and/or syrup and/or gel and/or cream and/or ointment and/or suppository and/or aerosol and/or drops.

The invention also includes lipooligoureas of general formula **(1)** for use as a medicament. Preferably, the lipooligoureas of general formula **(1)** are intended for the treatment of bacterial infections, preferably caused by gram-positive bacteria, especially of the Staphylococcus genus.

Lipooligoureas according to the invention can be obtained by any synthetic method known in the art. For example, said synthetic method may include either liquid-phase synthesis or solid-phase synthesis.

Lipooligoureas according to the invention have an amphiphilic structure, and due to the presence of urea residues of Lys^{u}, Dab^{u} or Arg^{u}, under physiological pH conditions (about 7.4) they have an excess positive charge, which facilitates their interaction with the bacterial cell membrane.

Lipooligoureas according to the invention, due to their growth inhibitory activity against strains of gram-positive bacteria such as *Enterococcus faecalis, Staphylococcus aureus, Staphylococcus epidermidis,* and gram-negative bacteria such as *Klebsiella pneumoniae, Pseudomonas aeruginosa, Yersinia enterocolitica,* can be used to treat bacterial infections. Lipooligoureas according to the invention show enhanced activity against *Staphylococcus* bacteria. Importantly, lipooligoureas according to the invention show significant growth inhibitory activity against strains of methicillin-resistant *Staphylococcus aureus* (MRSA) bacteria.

The advantage of lipooligoureas according to the invention is their mechanism of action, based on direct interaction with the bacterial cell membrane, which in turn reduces the risk of drug resistance. In addition, the described lipooligoureas according to the invention are relatively simple structures, which significantly facilitates their synthesis. Their adequate solubility in aqueous media is also ensured, which significantly broadens the range of possibilities regarding the application form of a potential pharmaceutical composition.

To illustrate the invention, an example is enclosed below, which, however, should not be interpreted in any way as limiting the scope of the invention.

### EXAMPLE

### Bacterial strains and culture media

All bacterial strains were obtained from the Polish Collection of Microorganisms (PCM) or American Type Culture Collection (ATCC). Gram-positive bacterial strains: *Staphylococcus aureus* ATCC 29213, *Staphylococcus epidermidis* ATCC 12228, *Enterococcus faecalis* ATCC 14506. Methicillin-resistant *Staphylococcus aureus* (MRSA) bacterial strains: *Staphylococcus aureus* 401/5, *Staphylococcus aureus* 4811/13, *Staphylococcus aureus* 5288/13. Gram-negative bacterial strains: *Escherichia coli* ST2-8624 O157:H7, *Pseudomonas aeruginosa* PAO1 PCM 499, *Klebsiella pneumoniae* PCM 1, *Salmonella sv. typhimurium* TT622, and *Yersinia enterocolitica* PCM 2081. All strains were cultured in LB medium (lysogenic broth).

### Synthesis of tested lipooligoureas according to the invention

Oligoureas were obtained from succinimidyl carbamates derivatives of 2-azidoethylamines substituted at position 2. These building blocks were obtained from amino acids according to a method known from the literature (C. Douat-Casassus, K. Pulka, P. Claudon and G. Guichard, Org. Lett., 2012, 14, 3130-3133). The oligoureas were prepared on a solid phase, on Rink amide resin, NovaPEG type with a loading of 0.45 mmol/g. Each time 300 mg of resin was used for synthesis. The synthesis was carried out under microwave irradiation, and each step was verified by a chloranil test. For the coupling step of individual building blocks, 1.5 eq of the respective block, 3 eq of DIPEA and 4 mL of DMF were used. The reaction was carried out in a microwave reactor under the following conditions: 50 °C and 50 W for 30 min. The step was repeated (1 or 2 times) until the appropriate color of the resin grains was obtained in the chloranil test. The step of reducing the azide group to the amine group was carried out with a 1 M solution of trimethylphosphine (PMe₃) in THF. 10 eq of PMe₃ was used, and the reaction was carried out in a 7:3 1,4-dioxane:H₂O solution. The reaction was carried out in a microwave reactor, under the same conditions as the coupling step. The reduction reaction was repeated until the appropriate color of the resin grains was obtained in the chloranil test. After the synthesis of the oligomeric portion, the carboxylic acid (FA) residue was attached. 3.5 eq of acid, 3.5 eq of HBTU and 9 eq of DIPEA were used. The reaction was carried out in DMF at room temperature for 24 h. The final compound was cleaved from the resin using a stripping mixture consisting of TFA:TIS:H₂O in a ratio of 95:2.5:2.5. Lipooligoureas were purified by semi-preparative HPLC using acetonitrile and water with 0.1% TFA as phases. A gradient was selected for each compound so as to obtain the best possible partitioning and yield. Oligoureas after HPLC purification were obtained as salts, trifluoroacetates. The trifluoroacetate ion was then exchanged for the chloride ion using a DOWEX 1X8-100 type ion exchange resin, and the final compounds were obtained after lyophilization of the solutions after ion exchange.

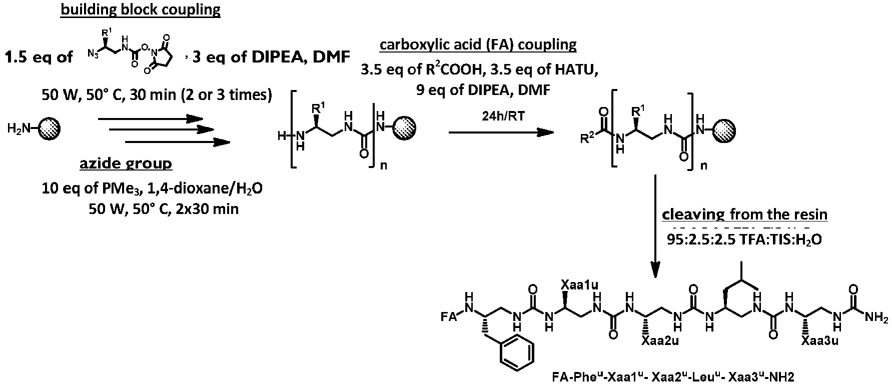

The following part of the example describes the biological activity of exemplary lipooligoureas with formulas:

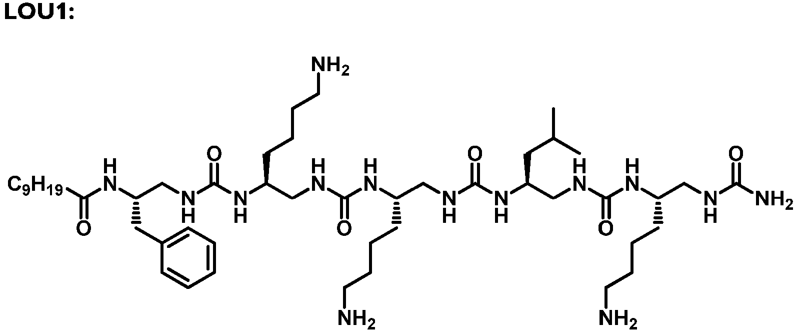

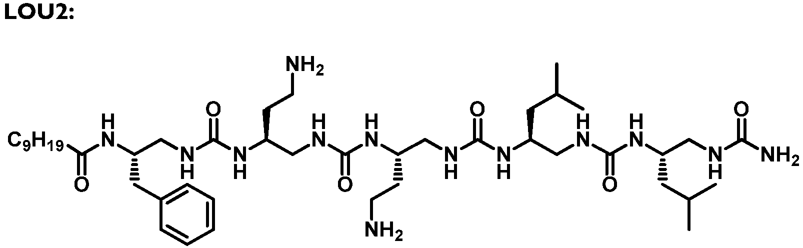

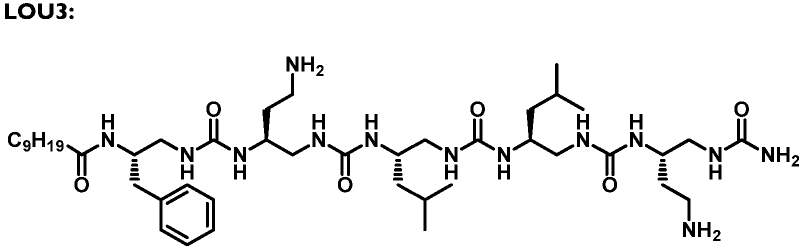

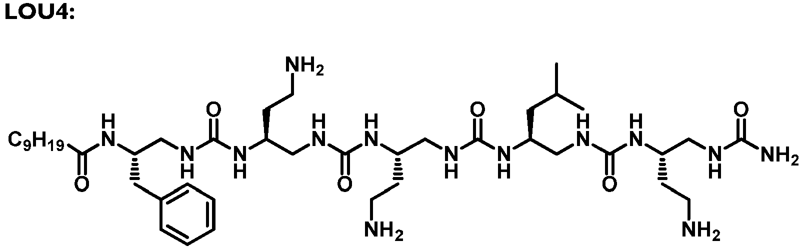

### Determination of the minimum inhibitory concentration (MIC)

10 mL of LB medium was inoculated with the material from a single bacterial colony and cultured at 30 °C with shaking overnight. The optical density of overnight cultures (at 600 nm) was then brought to 0.05 by dilution with a fresh portion of LB medium. The test compounds were dissolved in water. Serial dilutions of the compounds were performed in LB medium ranging from 5 to 50 µg/mL (final concentrations). The experiment was carried out by adding 100 µL of each prepared dilution to 100 µL of diluted overnight bacterial culture in the wells of a 96-well titer plate. The MIC was defined as the lowest concentration of the test compound needed to inhibit bacterial growth assessed after 24 h of incubation at 30 °C with shaking (final optical density at 600 nm no greater than 0.05). Optical density measurements were conducted using a TECAN Sunrise plate reader. Data were obtained from three independent experiments. The results obtained are summarized in Table 1.

**Table 1**

| **Bacterial strain** | **MIC [µg/m L = mg/L]** | | | |
|---|---|---|---|---|
| | **LOU1** | **LOU2** | **LOU3** | **LOU4** |
| (+) *Enterococcus faecalis* ATCC 14506 | n.d. | 20 | 5 | 40 |
| (-) *Escherichia coli* ST2-8624 O157:H7 | n.d. | 30 | 10 | n.d. |
| (-) *Klebsiella pneumoniae* PCM 1 | n.d. | 30 | 20 | n.d. |
| (-) *Pseudomonas aeruginosa* PAO1 PCM 499 | n.d. | 30 | 10 | n.d. |
| (-) *Salmonella sv. typhimurium* TT622 | n.d. | 30 | 20 | n.d. |
| (+) *Staphylococcus aureus* ATCC 29213 | 30 | 10 | 5 | 20 |
| (+) *Staphylococcus epidermidis* ATCC 12228 | 30 | 10 | 5 | 20 |
| (-) *Yersinia enterocolitica* PCM 2081 | n.d. | 30 | 10 | n.d. |
| **Methicillin-resistant *Staphylococcus aureus* (MRSA) strains** | | | | |
| (+) 401/5 | 30 | 10 | 5 | 20 |
| (+) 4811/13 | 30 | 10 | 5 | 30 |
| (+) 5288/13 | 40 | 10 | 5 | 30 |

| | | | | |
|---|---|---|---|---|
| MIC = minimum inhibitory concentration n.d. = not determined, i.e., the MIC value was higher than 50 µg/mL | | | | |

The results of the measurements summarized in Table 1 indicate that all of the lipooligoureas tested show varying activity against the bacterial strains tested. Lipooligoureas according to the invention show activity against both gram-positive and gram-negative bacterial strains. The results obtained indicate a certain degree of selectivity of the lipooligoureas according to the invention against gram-positive strains of *Staphylococcus aureus* ATCC 29213 and *Staphylococcus epidermidis* ATCC 12228, for which by far the highest activity of the tested lipooligoureas was observed, i.e. the lowest values of MIC. This trend is also reflected in the activity against strains of methicillin-resistant *Staphylococcus aureus* (MRSA). In this case, LOU2 and LOU3 show particularly high activity.

### Exemplary pharmaceutical composition according to the invention

Antibacterial ointment including in its composition:
- 40.0 g of aqueous 1% boric acid solution;
- 29.9 g of lanolin;
- 29.9 g of eucerin;
- 0.2 g of lipooligourea according to the invention.

### Susceptibility of lipooligoureas to proteolytic degradation

Stability studies of lipooligoureas in serum were performed on LOU1 - LOU4 compounds using human serum (obtained from Innovative Research, Inc., USA) without clotting factors. Lipooligourea stock solutions (0.168 M) were diluted (1:69) in human serum to an incubation concentration of 2.4 µmol/ml for each lipooligourea. Samples were then incubated at 37 °C and aliquots of 50 µL were taken at specified time intervals (0 min, 24 h, 48 h, 72 h, 96 h).

To precipitate serum proteins, 200 µL of ACN/H₂O/FA (89:10:1, v/v) was added to the collected samples, yielding turbid mixtures. These mixtures were vortexed at 3000 rpm for 1 minute, cooled in the refrigerator for 20 minutes, and then centrifuged at 4 °C (14,000 rpm) for 15 minutes to settle the proteins. 150 µL of supernatant was collected from each sample, and 300 µL of water was added before lyophilization. The lyophilized samples were then redissolved in 150 µL ACN/H₂O/FA (10:90:0.01, v/v) and analyzed using RP-HPLC, where a gradient of 10% solvent B in A for 5 minutes was used, followed by a gradient of 10% to 97% solvent B in A for 30 minutes, where solvent A is 0.1% TFA in H₂O, and solvent B is 0.1% TFA in CH₃CN. H-Trp-OH and Z-Lys-OH internal standards were used in the analysis. Two independent experiments were performed for each sample, with each experiment performed once.

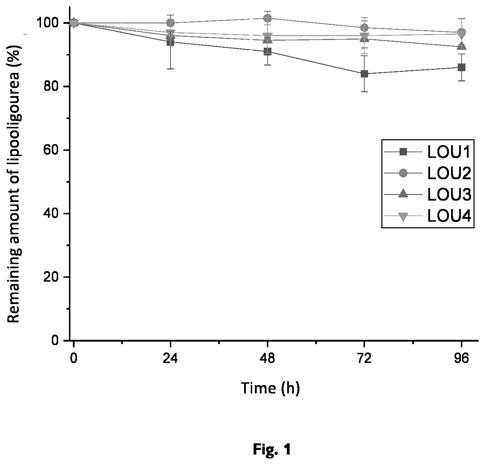

The susceptibility of LOU1 - LOU4 compounds to proteolytic degradation was assessed by incubating the lipooligoureas in human serum at 37 °C for a total time of 96 hours. The progress of enzymatic degradation was monitored for each sample at 24-hour intervals by RP-HPLC and changes in the lipooligourea peak area on the chromatogram were determined. The initial area at time t0 was taken as 100%. After 96 hours, the largest decrease (about 15%) in peak area was observed for LOU1. However, this decrease could potentially be attributed to co-precipitation of lipooligourea with serum proteins, as we did not detect any new peaks corresponding to proteolytic degradation products. In contrast, the peak areas for the other compounds showed no significant changes (variation of up to 8%), thus confirming the expected high resistance of lipooligoureas to proteolytic degradation in serum. Figure 1 shows the time-dependent changes in lipooligourea concentrations.

Fig. 1 shows time-dependent changes in the amount of remaining lipooligourea subjected to proteolytic degradation in human serum.

## Claims

1. A lipooligourea with general formula 1
**FA-Phe^{u}-Xaa1^{u}-Xaa2^{u}-Leu^{u}-Xaa3^{u}-NH₂** **(1)**
wherein
FA is a linear or branched carboxylic acid residue containing 6 to 15 carbon atoms in the alkyl chain;
Xaa1^{u} is either Lys^{u} or Dab^{u} or Arg^{u};
Xaa2^{u} is either Lys^{u} or Dab^{u} or Leu^{u} or Arg^{u};
Xaa3^{u} is either Lys^{u} or Dab^{u} or Leu^{u} or Arg^{u}.

2. The lipooligourea according to claim 1, selected from the group consisting of

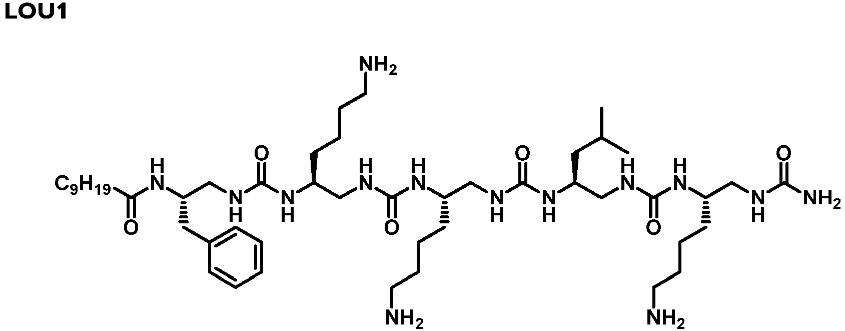

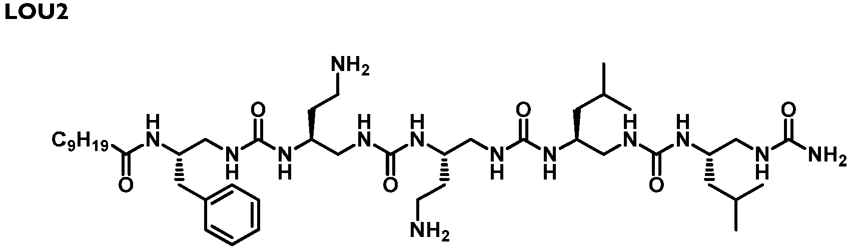

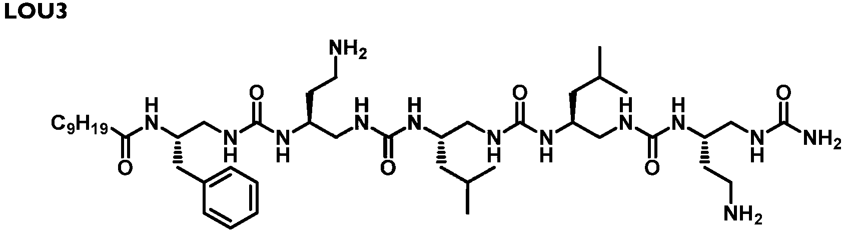

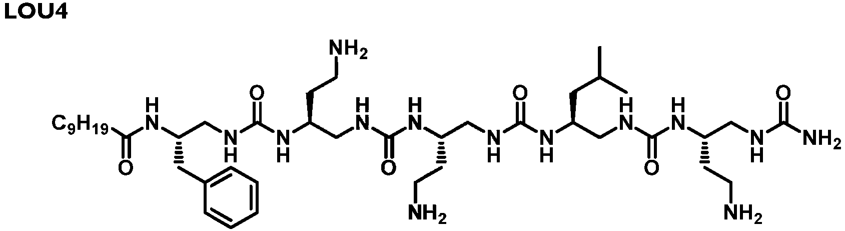

3. The lipooligourea as defined in claims 1 or 2 for use as a medicament.

4. The lipooligourea as defined in claims 1 or 2 for use in the treatment or prevention of bacterial infection.

5. The lipooligourea for use according to claim 4, **characterized in that** the bacterial infection is caused by either a gram-positive or a gram-negative bacterium.

6. The lipooligourea for use according to claims 4 or 5, **characterized in that** the bacterial infection is caused by a *Staphylococcus* bacterium.

7. The lipooligourea for use according to claims 4 or 5 or 6, **characterized in that** the bacterial infection is caused by *Staphylococcus aureus* or *Staphylococcus epidermidis.*

8. The lipooligourea for use according to claims 4 or 5, **characterized in that** the bacterial infection is caused by a strain of methicillin-resistant *Staphylococcus aureus* (MRSA).

9. Lipooligourea with formula LOU2 or LOU3 for use in the treatment or prevention of a bacterial infection caused by a strain of methicillin-resistant *Staphylococcus aureus* (MRSA).

10. A pharmaceutical composition, **characterized in that** it contains a lipooligourea of general formula 1
**FA-Phe^{u}-Xaa1^{u}-Xaa2^{u}-Leu^{u}-Xaa3^{u}-NH₂** **(1)**
wherein
FA is a linear or branched carboxylic acid residue containing 6 to 15 carbon atoms in the alkyl chain;
Xaa1^{u} is either Lys^{u} or Dab^{u} or Arg^{u};
Xaa2^{u} is either Lys^{u} or Dab^{u} or Leu^{u} or Arg^{u};
Xaa3^{u} is either Lys^{u} or Dab^{u} or Leu^{u} or Arg^{u}.

11. The pharmaceutical composition according to claim 10, **characterized in that** it contains a lipooligourea selected from the group consisting of

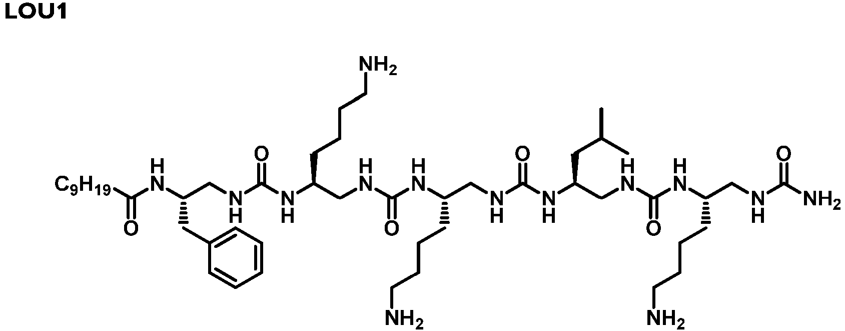

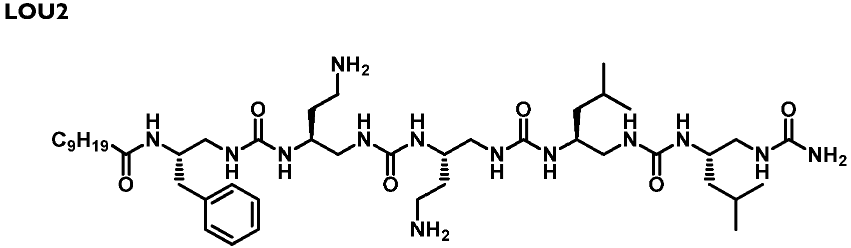

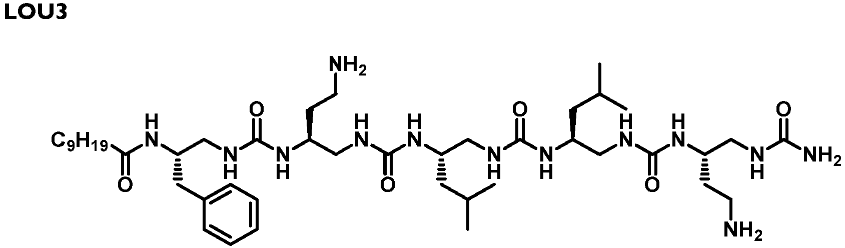

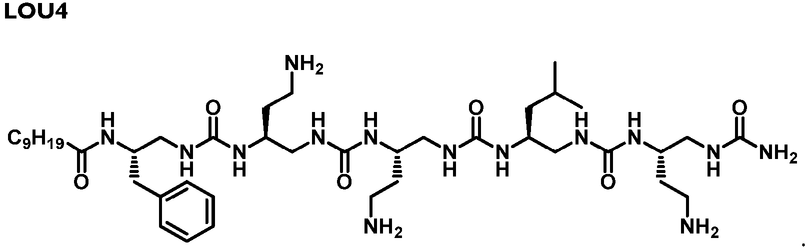

12. The pharmaceutical composition according to claims 10 or 11, **characterized in that** it contains 0.1 to 99% by weight of at least one lipooligourea.

13. The pharmaceutical composition according to any of claims 10 to 12, **characterized in that** it contains a pharmaceutically acceptable carrier and/or diluent and/or aid and/or excipient.

14. The pharmaceutical composition according to claim 13, **characterized in that** it contains corn starch and/or gelatin and/or lactose and/or sucrose and/or microcrystalline cellulose and/or kaolin and/or mannitol and/or dicalcium phosphate and/or sodium chloride and/or alginic acid.

15. The pharmaceutical composition according to any of the claims 10 to 14, **characterized in that** it is in a form suitable for oral and/or intravenous and/or intramuscular and/or subcutaneous and/or parenteral and/or rectal and/or topical administration.

16. The pharmaceutical composition according to any of the claims 10 to 15, **characterized in that** it is in the form of a tablet and/or capsule and/or elixir and/or suspension and/or syrup and/or gel and/or cream and/or ointment and/or suppository and/or aerosol and/or drops.

17. The pharmaceutical composition according to any of the claims 10 to 16, **characterized in that** it further contains at least one other antibacterial agent.

## Patentansprüche

1. Lipooligoharnstoff mit der allgemeinen Formel 1
**FA-Phe^{u}-Xaal ^{u}-Xaa2^{u}-Leu^{u}-Xaa3^{u}-NH₂** **(1)**
wobei
FA ein linearer oder verzweigte Carboxylsäurerest enthaltend 6 bis 15 Kohlenstoffatome in der Alkylkette ist,
Xaa1^{u} entweder Lys^{u} oder Dab^{u} oder Arg^{u} ist;
Xaa2^{u} entweder Lys^{u} oder Dab^{u} oder Leu^{u} oder Arg^{u} ist;
Xaa3^{u} entweder Lys^{u} oder Dab^{u} oder Leu^{u} oder Arg^{u} ist.

2. Lipooligoharnstoff nach Anspruch 1, ausgewählt aus der Gruppe bestehend aus

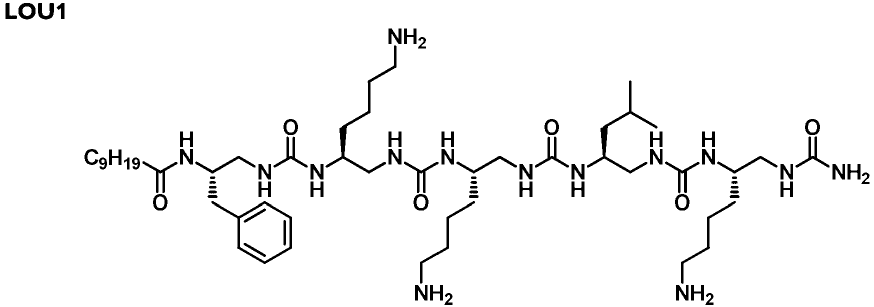

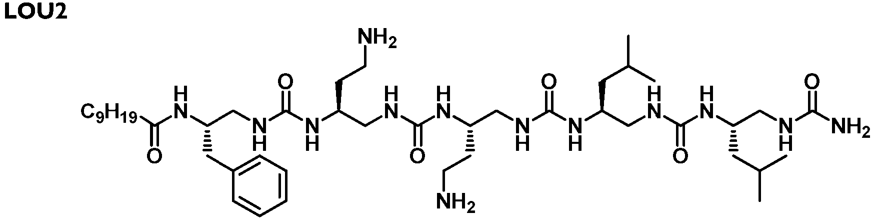

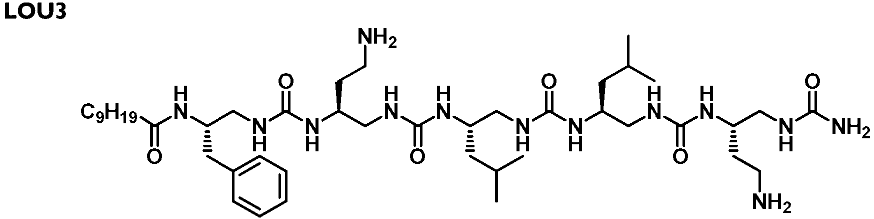

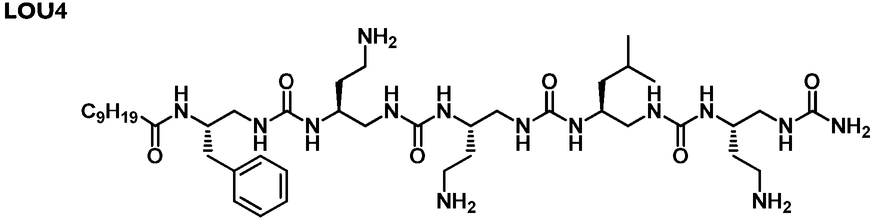

3. Lipooligoharnstoff wie in Anspruch 1 oder 2 definiert zur Verwendung als Arzneistoff.

4. Lipooligoharnstoff wie in Anspruch 1 oder 2 definiert zur Verwendung bei der Behandlung oder Vorbeugung bakterieller Infektionen.

5. Lipooligoharnstoff zur Verwendung nach Anspruch 4, **dadurch gekennzeichnet, dass** die bakterielle Infektion durch entweder ein grampositives Bakterium oder ein gramnegatives Bakterium verursacht ist.

6. Lipooligoharnstoff zur Verwendung nach Anspruch 4 oder 5, **dadurch gekennzeichnet, dass** die bakterielle Infektion durch ein Staphylococcus-Bakterium verursacht ist.

7. Lipooligoharnstoff zur Verwendung nach Anspruch 4 oder 5 oder 6, **dadurch gekennzeichnet, dass** die bakterielle Infektion durch *Staphylococcus aureus* oder *Staphylococcus epidermidis* verursacht ist.

8. Lipooligoharnstoff zur Verwendung nach Anspruch 4 oder 5, **dadurch gekennzeichnet, dass** die bakterielle Infektion durch einen Stamm von gegen Methicillin resistentem *Staphylococcus aureus* (MRSA) verursacht ist.

9. Lipooligoharnstoff mit der Formel LOU2 oder LOU3 zur Verwendung bei der Behandlung oder Vorbeugung einer bakteriellen Infektionen verursacht durch einen Stamm von gegen Methicillin resistentem *Staphylococcus aureus* (MRSA).

10. Pharmazeutische Zusammensetzung, **dadurch gekennzeichnet, dass** sie einen Lipooligoharnstoff der allgemeinen Formel 1
**FA-Phe^{u}-Xaa1^{u}-Xaa2^{u}-Leu^{u}-Xaa3^{u}-NH₂** **(1)**
enthält, wobei
FA ein linearer oder verzweigte Carboxylsäurerest enthaltend 6 bis 15 Kohlenstoffatome in der Alkylkette ist,
Xaa1^{u} entweder Lys^{u} oder Dab^{u} oder Arg^{u} ist;
Xaa2^{u} entweder Lys^{u} oder Dab^{u} oder Leu^{u} oder Arg^{u} ist;
Xaa3^{u} entweder Lys^{u} oder Dab^{u} oder Leu^{u} oder Arg^{u} ist.

11. Pharmazeutische Zusammensetzung nach Anspruch 10, **dadurch gekennzeichnet, dass** sie einen Lipooligoharnstoff ausgewählt aus der Gruppe bestehend aus 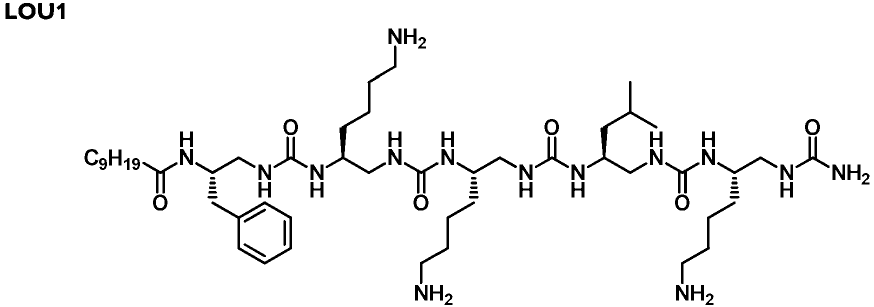 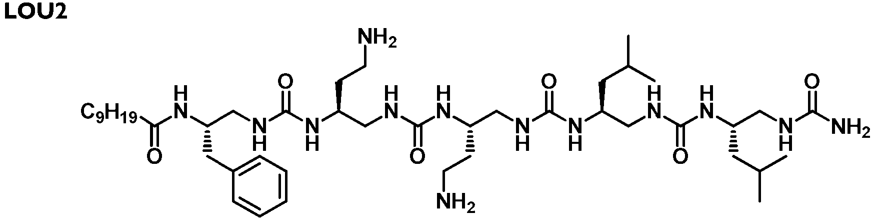 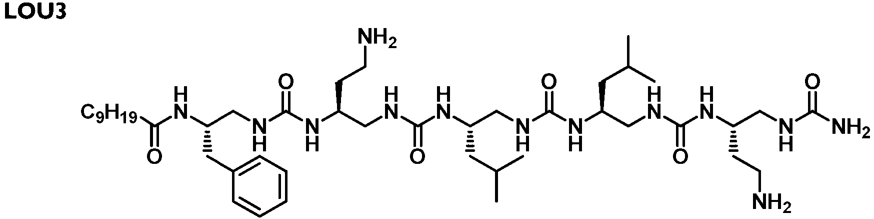 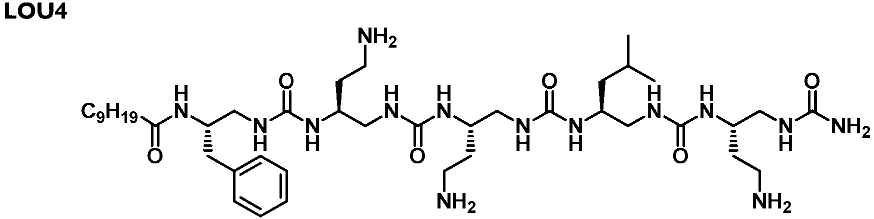 enthält.

12. Pharmazeutische Zusammensetzung nach Anspruch 10 oder 11, **dadurch gekennzeichnet, dass** sie 0,1 bis 99 Gew.-% zumindest eines Lipooligoharnstoffes enthält.

13. Pharmazeutische Zusammensetzung nach einem der Ansprüche 10 bis 12, **dadurch gekennzeichnet, dass** sie einen pharmazeutisch akzeptablen Träger und/oder Verdünner und/oder Hilfsstoff und/oder Exzipienten enthält.

14. Pharmazeutische Zusammensetzung nach Anspruch 13, **dadurch gekennzeichnet, dass** sie Maisstärke und/oder Gelatine und/oder Laktose und/oder Saccharose und/oder mikrokristalline Zellulose und/oder Kaolin und/oder Mannit und/oder Dikalziumphosphat und/oder Natriumchlorid und/oder Alginsäure enthält.

15. Pharmazeutische Zusammensetzung nach einem der Ansprüche 10 bis 14, **dadurch gekennzeichnet, dass** sie in einer Form vorliegt, die zur oralen und/oder intravenösen und/oder intramuskulären und/oder subkutanen und/oder parenteralen und/oder rektalen und/oder topischen Verabreichung geeignet ist.

16. Pharmazeutische Zusammensetzung nach einem der Ansprüche 10 bis 15, **dadurch gekennzeichnet, dass** sie in Form einer Tablette und/oder Kapsel und/oder Heiltranks und/oder Suspension und/oder Sirups und/oder Gels und/oder Creme und/oder Salbe und/oder Zäpfchens und/oder Aerosols und/oder von Tropfen vorliegt.

17. Pharmazeutische Zusammensetzung nach einem der Ansprüche 10 bis 16, **dadurch gekennzeichnet, dass** sie zudem zumindest einen anderen antibakteriellen Wirkstoff enthält.

## Revendications

1. Lipooligourée de formule générale 1
**FA-Phe^{u}-Xaa1^{u}-Xaa2^{u}-Leu^{u}-Xaa3^{u}-NH₂** **(1)**
dans laquelle
FA est un résidu d'acide carboxylique linéaire ou ramifié contenant 6 à 15 atomes de carbone dans la chaîne alkyle ;
Xaa1^{u} est soit Lys^{u} soit Dab^{u} soit Arg^{u} ;
Xaa2^{u} est soit Lys^{u} soit Dab^{u} soit Leu^{u} soit Arg^{u} ;
Xaa3^{u} est soit Lys^{u} soit Dab^{u} soit Leu^{u} soit Arg^{u}.

2. Lipooligourée selon la revendication 1, choisie dans le groupe consistant en :

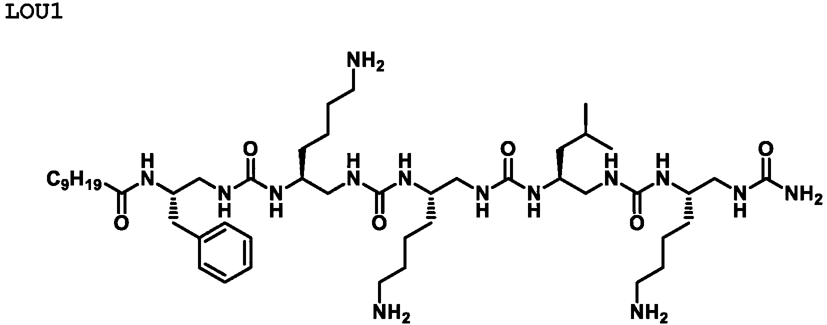

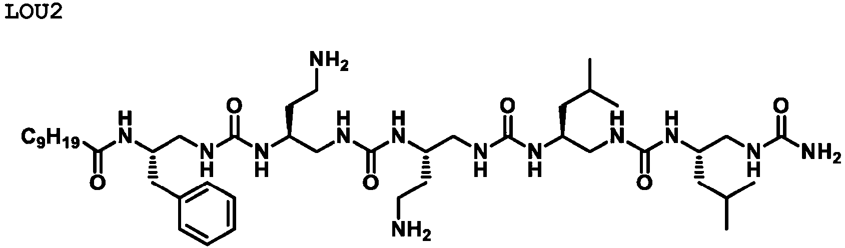

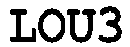

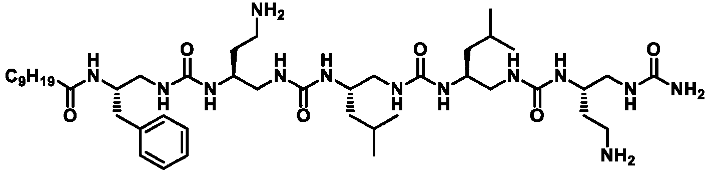

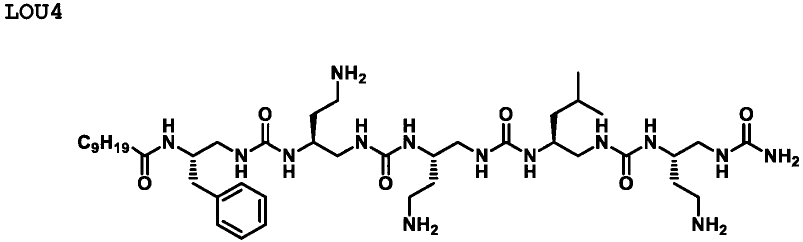

3. Lipooligourée selon les revendications 1 ou 2 pour une utilisation comme médicament.

4. Lipooligourée selon les revendications 1 ou 2 pour une utilisation dans le traitement ou la prévention d'une infection bactérienne.

5. Lipooligourée pour une utilisation selon la revendication 4, **caractérisée en ce que** l'infection bactérienne est causée par une bactérie à Gram positif ou à Gram négatif.

6. Lipooligourée pour une utilisation selon les revendications 4 ou 5, **caractérisée en ce que** l'infection bactérienne est causée par une bactérie *Staphylococcus.*

7. Lipooligourée pour une utilisation selon les revendications 4 ou 5 ou 6, **caractérisée en ce que** l'infection bactérienne est causée par *Staphylococcus aureus* ou *Staphylococcus epidermidis.*

8. Lipooligourée pour une utilisation selon les revendications 4 ou 5, **caractérisée en ce que** l'infection bactérienne est causée par une souche de *Staphylococcus aureus* résistante à la méthicilline (SARM).

9. Lipooligourée de formule LOU2 ou LOU3 pour une utilisation dans le traitement ou la prévention d'une infection bactérienne causée par une souche de *Staphylococcus aureus* résistante à la méthicilline (SARM).

10. Composition pharmaceutique, **caractérisée en ce qu'**elle contient une lipooligourée de formule générale 1
**FA-Phe^{u}-Xaa1^{u}-Xaa2^{u}-Leu^{u}-Xaa3^{u}-NH₂** **(1)**
dans laquelle
FA est un résidu d'acide carboxylique linéaire ou ramifié contenant 6 à 15 atomes de carbone dans la chaîne alkyle ;
Xaa1^{u} est soit Lys^{u} soit Dab^{u} soit Arg^{u} ;
Xaa2^{u} est soit Lys^{u} soit Dab^{u} soit Leu^{u} soit Arg^{u} ;
Xaa3^{u} est soit Lys^{u} soit Dab^{u} soit Leu^{u} soit Arg^{u}.

11. Composition pharmaceutique selon la revendication 10, **caractérisée en ce qu'**elle contient une lipooligourée choisie dans le groupe consistant en

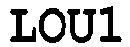

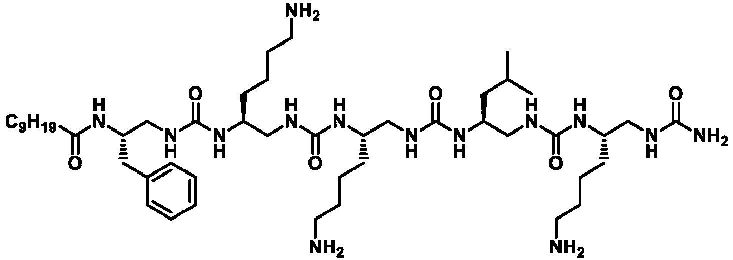

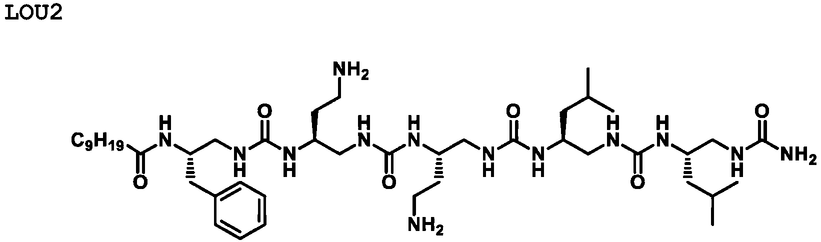

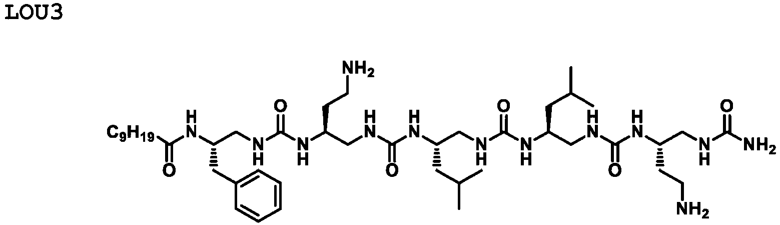

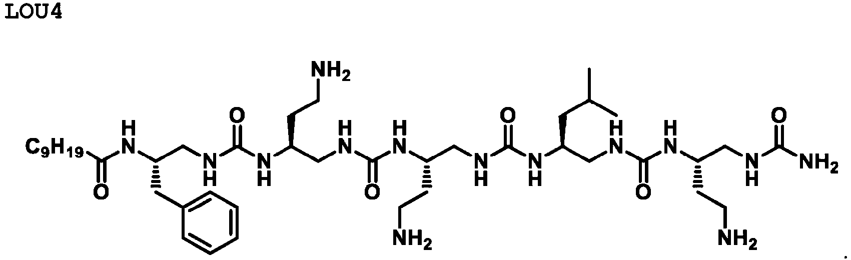

12. Composition pharmaceutique selon les revendications 10 ou 11, **caractérisée en ce qu'**elle contient 0,1 à 99 % en poids d'au moins une lipooligourée.

13. Composition pharmaceutique selon quelconque des revendications 10 à 12, **caractérisée en ce qu'**elle contient un support et/ou un diluant et/ou un auxiliaire et/ou un excipient pharmaceutiquement acceptables.

14. Composition pharmaceutique selon la revendication 13, **caractérisée en ce qu'**elle contient de l'amidon de maïs et/ou de la gélatine et/ou du lactose et/ou du saccharose et/ou de la cellulose microcristalline et/ou du kaolin et/ou du mannitol et/ou du phosphate dicalcique et/ou du chlorure de sodium et/ou de l'acide alginique.

15. Composition pharmaceutique selon quelconque des revendications 10 à 14, **caractérisée en ce qu'**elle se présente sous une forme adaptée à une administration orale et/ou intraveineuse et/ou intramusculaire et/ou sous-cutanée et/ou parentérale et/ou rectale et/ou topique.

16. Composition pharmaceutique selon quelconque des revendications 10 à 15, **caractérisée en ce qu'**elle se présente sous la forme d'un comprimé et/ou d'une capsule et/ou d'un élixir et/ou d'une suspension et/ou d'un sirop et/ou d'un gel et/ou d'une crème et/ou d'une pommade et/ou d'un suppositoire et/ou d'un aérosol et/ou de gouttes.

17. Composition pharmaceutique selon quelconque des revendications 10 à 16, **caractérisée en ce qu'**elle contient en outre au moins un autre agent antibactérien.
